# EUROPEAN PATENT SPECIFICATION

(11) **EP 1 724 203 B1**
(45) Date of publication and mention of the grant of the patent: **01.02.2012**
(21) Application number: 05720346.5
(22) Date of filing: 09.03.2005
(51) Int. Cl.: B65D 47/36, B65D 47/20, A61J 1/05

(54) **EYEDROPPER**
AUGENTROPFENFLASCHE
FLACON COMPTE-GOUTTES OCULAIRE

(30) Priority: 09.03.2004 JP 2004065543
(43) Date of publication of application: 22.11.2006
(73) Proprietor: SANTEN PHARMACEUTICAL CO., LTD., Higashiyodogawa-ku, Osaka-shi, Osaka 533-8651 (JP)
(72) Inventor: YAMADA, Hiroshi, c/o SANTEN PHARMACEUTICAL CO, LTD, Osaka-shi, Osaka 5338651 (JP); MIYOSHI, Naohito c/o SANTEN PHARMACEUTICAL CO, LTD, Osaka-shi, Osaka 5338651 (JP)
(74) Representative: Lemcke, Brommer & Partner
(86) International application number: PCT/JP2005/004074
(87) International publication number: WO 2005/085086

(56) References cited:
- WO-A1-01/74291
- WO-A1-02/00160
- JP-A- 61 060 457
- JP-A- 2001 187 112
- JP-U- 5 053 648
- JP-Y2- 6 009 977
- US-A1- 2002 153 386

## Description

### TECHNICAL FIELD

The present invention relates to an eye dropper comprising a container body and an instilling opening member.

### BACKGROUND ART

It has conventionally been proposed to provide a sealing portion for a container body to block the interior of the container from ambient air in order to maintain a sterile condition of liquid contained in the container body and preserve the sterile liquid for an extended time. However, even if the sealing portion is provided, ambient air flows into the container body or liquid exposed to ambient air flows back into the container body when the sealing portion is broken, as in the case of ordinary containers. Thus, when the incoming ambient air or the backflow liquid mixes with the liquid within the container body, the entire liquid contained in the container body can be contaminated by microbes or the like present in the air or the liquid.
The container is an eyedrops container and an instilling opening of the eyedrops container may sometimes contact tears or eyelashes in time of instillation to be contaminated by microbes or the like. As a result, when the liquid having contacted tears or eyelashes flows back into the container body, the entire liquid contained in the container body will be contaminated and the sterile condition in the interior of the container body cannot be maintained. In addition, once microbes or the like flow into the container body, the microbial contamination is likely to spread over time.

On the other hand, a preservative can be added to the liquid prior to being stored in the container body to cope with the situation. However, it is desirable to avoid addition of the preservative as much as possible, taking influences of the preservative on human bodies into consideration.

In view of the above, various types of liquid containers have been proposed for the purpose of preventing the backflow into the container body. For instance, one proposal is to provide a check valve function for the sealing portion (Patent Document 1), and another proposal is to provide a check valve for preventing liquid having flowed from the sealing portion to the instilling opening member from flowing back into the container body (Patent Document 2).
Patent Document 1: Japanese Patent Publication "Kokai" No. 2003-169839
Patent Document 2: Japanese Patent Publication "Kokai" No. 2003-26250

### DISCLOSURE OF THE INVENTION

### PROBLEM TO BE SOLVED BY THE INVENTION

However, according to Patent Document 1, the sealing portion is formed of a check valve made of an elastic member with an openable and closable pinhole. Thus, in order to open or close the check valve, a communicating needle is required which is movable from the instilling opening to the container body. However, the communicating needle is likely to contact ambient air as positioned close to the instilling opening of the check valve. The communicating needle having contacted ambient air is subsequently moved from the instilling opening toward the container body, and thus a possibility of contaminating the interior of the container body.

According to Patent Document 2, liquid having been contaminated adjacent the instilling opening can, prima facie, be prevented from flowing back toward the container body beyond the check valve. However, since the check valve is provided closer to the instilling opening than the sealing portion, it is necessary to form the sealing portion and the check valve separately to be separable from each other so that the sealing portion may be opened. This arrangement creates a possibility that the interior of the container body is contaminated since the sealing portion is opened with the unsealing portion (the member 10 in Patent Document 2) being exposed to ambient air.
When opening the sealing portion, the user of the liquid container may inadvertently separate the sealing portion and check valve. Then, the liquid inside the container body and in the vicinity of the sealing portion of the container body will be exposed to ambient air.

The object of the present invention is to provide a liquid container in which liquid has a reduced chance of becoming contaminated.

### MEANS FOR SOLVING THE PROBLEM

A characteristic construction of an eye dropper according to the present invention is disclosed in the appending main claim.

With the above-noted construction, both the unsealing member and the sealing portion are positioned closer to an instilling opening formed in the instilling opening member than the check valve. Therefore, an opening operation can be executed without operating a portion between the check valve and the container body in breaking the sealing portion with the unsealing member by pushing in the instilling opening member toward the container body. This substantially eliminates the possibility of spreading contamination over the interior of the container body through the check valve in the opening process.

That is, the liquid may be maintained sterile for a long time without adding any chemicals such as a preservative thereto.

The instilling opening member in the above-noted construction is shiftable between a first position to maintain the unsealing member not piercing the sealing portion and a second position to allow the unsealing member to pierce the sealing portion relative to the container body.

More particularly, when the unsealing member is in the position not to pierce the sealing portion, the sealing portion can maintain the sealed state, and thus the liquid within the container body can be maintained in an uncontaminated state. When the unsealing member pierces the sealing portion, the sealed state is canceled to allow the liquid to be used. Since the position shifting operation is carried out by pushing in the instilling opening member toward the container body, the position can be shifted without exposing the sealing portion to the outside. This can reduce the possibility of contamination occurring in the space within the instilling opening member or adjacent the sealing portion.

In the above-noted construction, the unsealing member may pierce the sealing portion from the container body toward the instilling opening member, or may pierce the sealing portion from the instilling opening member toward the container body.

According to the above construction, the unsealing member is provided at the side of the container body while the sealing portion is provided at the side of the instilling opening member, whereby the unsealing member is shifted from the first position spaced from or contacting the sealing portion to the second position to pierce the sealing portion by pushing in the instilling opening member to the container body. In the latter case as well, the construction acts in a similar way by swapping the positions of the sealing portion and unsealing member.
After the unsealing member pierces the sealing portion, the sealed condition of the sealing portion is cancelled to allow the liquid within the container body to move toward the instilling opening formed in the instilling opening member through the sealing portion. At this time, since the check valve is not affected by the sealing portion or the unsealing member, the liquid having reached the sealing portion does not flow back into the container body through the check valve, and also ambient air does not flow into the container body. Thus, the sterile condition can be maintained within the container body.

In employing such a construction, the unsealing member and the sealing portion may be distributed to the instilling opening member and the container body, respectively, or may be provided together on either side of the instilling opening member and the container body. Further, the instilling opening member may act as the unsealing member.

It is preferable that the container body has a flexible portion for varying the capacity of the container body in response to an amount of liquid contained in the interior of the container body.

When the check valve prevents the backflow of the liquid having reached adjacent the sealing portion or the inflow of the air present in the instilling opening member into the container body, the amount of the liquid within the container body is reduced, which results in a decreased inner pressure.
When the liquid is reduced by a small amount, the inner pressure is slightly decreased within the container body thereby to allow the check valve to fully perform its function. However, when a large amount of liquid is reduced, a force to draw the liquid toward the interior of the container body is produced in response to the decrease of the inner pressure of the container body, if the container body has excellent shape-memory properties. As a result, the check valve may not function, or a greater force may be required for discharging the liquid from the container body.
In order to avoid such problems, the container is provided with the flexible portion (a bellows-like container, for example) so that the capacity of the interior of the container body may vary in response to variations of the amount of the liquid present in the container body, thereby to constantly maintain the inner pressure substantially at the same level as atmospheric pressure. Therefore, the check valve functions properly, and the liquid is easily instilled from the container.

### BEST MODE FOR CARRYING OUT THE INVENTION

Embodiments of a liquid container according to the present invention will be described hereinafter with reference to the drawings.

### Embodiment 1

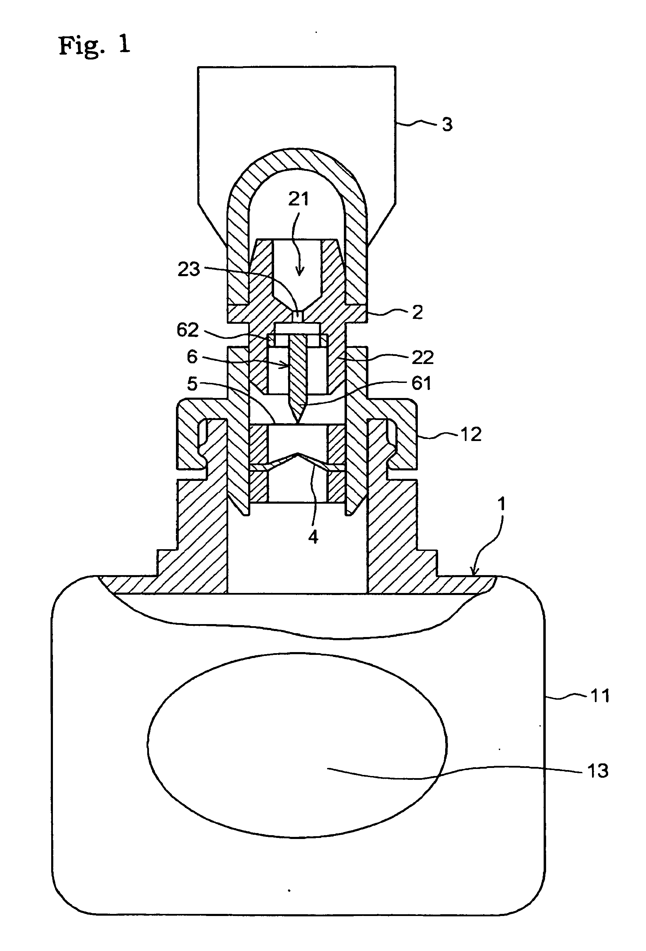

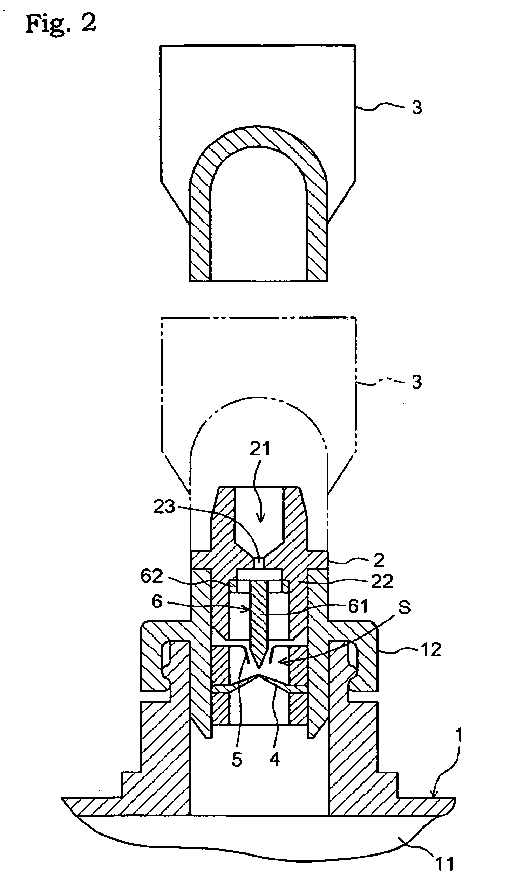

As shown in Figs. 1 and 2, the liquid container according to the present invention includes a container body 1 and an instilling opening member 2 screwed thereto.

The container body 1 includes a barrel 11 made of a resin forming a liquid storage space for containing mainly a liquid therein, and a cylindrical plug 12 for guiding the liquid such as medical liquid for eyedrops from the barrel 11 to outside of the container body. The barrel 11 and the plug 12 are formed separately from each other and integrated together to constitute the container body 1. The barrel 11 includes a thin-walled flexible portion 13.

The instilling opening member 2 is shaped cylindrical so that a cap 3 can be fitted thereon, and includes an instilling opening 21 formed at one end thereof, and a screw portion 22 formed at the other end thereof to be screwed to the plug 12 of the container body 1. A constricted portion 23 is formed in the interior of the instilling opening member 2 for restricting a flow rate of the liquid. The instilling opening 21 is formed to distil a fixed amount of the medical liquid maintained in the interior of the instilling opening member 2 extending from the constricted portion 23 to the one end thereof.

A check valve 4 is fitted in the plug 12 of the container body 1 for preventing the medical liquid having flowed out of the barrel 11 of the container body 1 toward the instilling opening member 2 from flowing back into the container body 1. A sealing portion 5 made of a resin film is provided for the check valve 4 close to the instilling opening member 2 for hermetically confining the medical liquid within the container body 1. The resin film is desirably broken and opened entirely and widely as a middle portion thereof is ruptured.

The instilling opening member 2 includes an unsealing member 6 therein having a needle portion 61 acting as an unsealing portion. The unsealing member 6 is arranged and fixed so as to move toward and penetrate the sealing portion 5 as the instilling opening member 2 is screwed into the plug 12.
The unsealing member 6 has a seat portion 62 fitted in the screw portion 22 of the instilling opening member 2. The needle portion 61 is mounted upright on the seat portion 62. This allows the unsealing member 6 to accommodate and maintain the needle portion 61 in a position extending downward along the axis of the instilling opening member 2 with the seat portion 62 fitted in the instilling opening member 2.
The seat portion 62 has a bore formed therein to facilitate passage of the liquid from the container body to the instilling opening.

The instilling opening member 2 with the cap 3 screwed thereto is kept in store as partly screwed into the plug 12. In this state, the unsealing member 6 is in a first position or non-piercing state spaced from or contacting the sealing portion 5 (see Fig. 1).
In starting to use this liquid container, the instilling opening member 2 is screwed into the plug 12 to move the needle portion 61 of the unsealing member 6 toward the container body 1. As a result, the unsealing member 6 is shifted to a second position to pierce the sealing portion 5 within the plug 12 (see Fig. 2).
Thus, the liquid within the container body 1 can be supplied from the sealing portion 5 through the check valve 4 and through the constricted portion 23 of the instilling opening member 2 to the instilling opening 21 to be distilled. At this time, the liquid remains in a space S defined between the constricted portion 23 and the check valve 4, but does not flow back to the container body 1. Also, ambient air does not flow into the container body 1.
Since the container body 1 includes the thin-walled flexible portion 13 which is easily deformable, the capacity of the interior of the container body 1 varies with variations in the amount of liquid in the container body 1 to constantly maintain an inner pressure at substantially the same level as atmospheric pressure. Therefore, air is not drawn from the instilling opening 21 to the interior of the container body 1.

### Embodiment 2

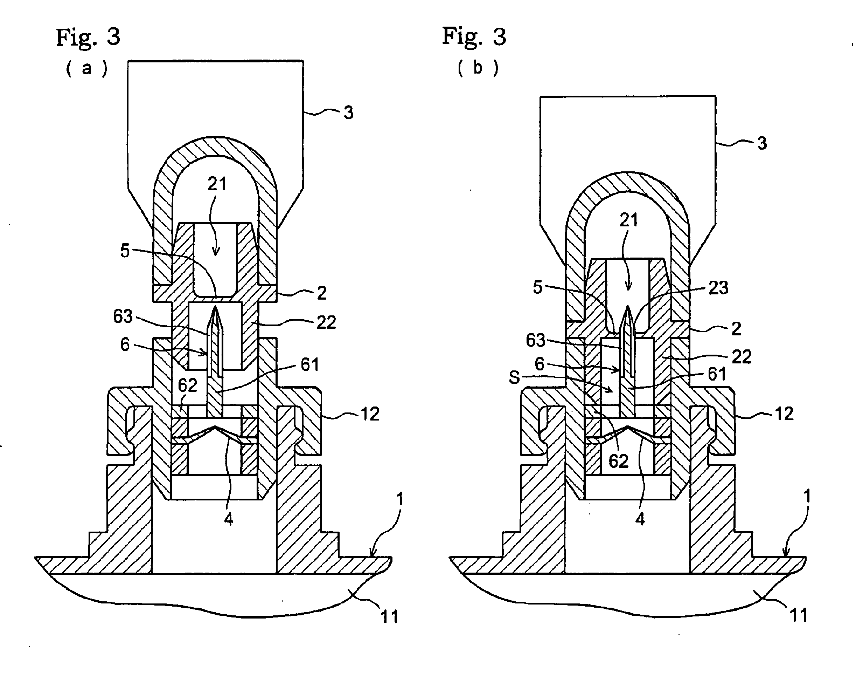

The sealing portion 5 and the unsealing member 6 in the above-noted embodiment may be arranged as shown in Fig. 3.

More particularly, in Fig. 3, the unsealing member 6 is fitted into the plug 12 of the container body 1, instead of being fitted into the instilling opening member 2, and arranged so that the needle portion 61 is directed to the instilling opening 21 along the axis of the instilling opening member 2. Further, the constricted portion 23 is provided in an unopened state to act as the sealing portion 5.

With this construction, the unsealing member 6 assumes the first position or non-piercing state spaced from or contacting the sealing portion 5 in the instilling opening member 2 in the stored position where the instilling opening member 2 is partly screwed into the plug 12 (see Fig. 3(a)).
In starting to use this liquid container, the instilling opening member 2 is screwed into the plug 12 to move the sealing portion 5 toward the container body 1. As a result, the needle portion of the unsealing member 6 is shifted to the second position to pierce the sealing portion 5 (see Fig. 3(b)).
At this time, the needle portion 61 is rotated about the axis to pierce the sealing portion 5, and thus may easily open the constricted portion 23 even if the instilling opening member 2 is made of a somewhat rigid material. Therefore, this arrangement is advantageous in that the portion of the sealing portion 5 that is to be formed as the constricted portion 23 and easily exposed to outside when the cap 3 is removed is not easily opened.
In order to use the container with the needle portion 61 piercing the sealing portion 5 and extending through the constricted portion 23, a groove portion 63 is preferably formed in an outer surface of the needle portion 61 along the axial direction to allow the liquid to pass through the constricted portion 23 by way of the groove portion 63.

Various types of conventional check valves may be used instead of the check valve 4 in the above-noted embodiments as long as the liquid is prevented from flowing back into the container body 1.

The unsealing member 6 may have a push-in blade instead of the needle portion 61. Further, it may be maintained in an inclined position instead of the position along the axial direction of the instilling opening member 2 and the plug 12 as long as the sealing portion 5 can be opened as the instilling opening member 2 is pushed in.

Further, in pushing in and opening the instilling opening member 2, the member 2 may be simply pressed into the plug 12, instead of forming the portion 22 thereon to be screwed to the plug 12.

As the liquid, various types of liquids other than the medical liquid for eyedrops are applicable.

In forming the flexible portion 13, various modifications are possible such as the container body 1 having a bellows-like portion in part or as a whole, instead of the container body 1 having a partly thinned portion.
On the other hand, the container body 1 may be formed as a container having a cylinder piston, a container having a check valve provided in the bottom, in which the capacity of the interior of the container body 1 may change in response to variations of the liquid amount in the container body thereby to constantly maintain the inner pressure at substantially the same level as atmospheric pressure.

Further, it is desirable to form the portions where the liquid adheres or remains (the instilling opening member 2, check valve 4, unsealing member 6 and plug 12, for example) of an antibacterial resin in order to restrain the liquid contained in the container body 1 from being contaminated by various microbes. As the antibacterial resin, a resin base having an antibacterial substance mixed thereto is generally used.

It is also possible to enhance the antibacterial effect by bringing the liquid moving from the space S defined between the constricted portion 23 and the check valve 4 to the instilling opening 21, into contact with a porous material having an antibacterial effect. Either of organic and inorganic filters may be employed as the porous material, and more particularly, any filters made of non-woven fabric, sponge (pumice) and cotton may be used and fitted on the instilling opening 21, for example.

As noted above, the liquid container of the invention may enhance the preservability of the liquid to allow the liquid to be used in a reliable and uncontaminated condition, thereby to improve handling convenience of the liquid.

### INDUSTRIAL UTILITY

The liquid container according to the present invention may be used as an eyedropper or the like used mainly for medical purpose.

### BRIEF DESCRIPTION OF DRAWINGS

[Fig. 1]
   Side view in vertical section of a liquid container in a stored condition according to Embodiment 1 of the present invention.
[Fig. 2]
   Side view in vertical section of the liquid container in a start-to-use condition according to Embodiment 1 of the present invention.
[Fig. 3]
   Side view in vertical section of a liquid container according to Embodiment 2 of the present invention.

### DESCRIPTION OF THE REFERENCE SIGNS

- 1: container body
- 2: instilling opening member
- 4: check valve
- 5: sealing portion
- 6: unsealing member
- 13: flexible portion

## Claims

1. An eyedropper comprising a container body (1) including a liquid storage space formed therein, a plug (12) for guiding a liquid to outside of the container body (1) and an instilling opening member (2) fitted into the plug (12) and formed to distil a fixed amount of medical liquid retained in the interior of said instilling opening member (2) through an instilling opening (21) formed at one end of the instilling opening member (2) and which is shaped cylindrical and has a cap (3) fitted thereon,
wherein the eyedropper further comprises a sealing portion (5) for hermetically confining liquid in the interior of the container body (1), an unsealing member (6) for opening the sealing portion (5), and a check valve (4) provided in the plug (12) and at a portion closer to the liquid storage space than the sealing portion (5) and the unsealing member (6) for allowing the unsealing member (6) to open the sealing portion (5) when the cap (3) is depressed along an axial direction of the container to push the instilling opening member (2) toward the container body(1), and to move the instilling opening member (2) relative to the plug (12), while preventing the liquid from flowing back to the interior of the container body (1).

2. The eyedropper defined in Claim 1, wherein the unsealing member (6) pierces the sealing portion (5) from the container body (1) toward the instilling opening member (2).

3. The eyedropper defined in Claim 1, wherein the unsealing member (6) pierces the sealing portion (5) from the instilling opening member (2) toward the container body (1).

4. The eyedropper defined in any one of Claims 1 through 3, wherein the container body (1) has a flexible portion (13) for varying the capacity of the container body (1) in response to an amount of liquid contained in the interior of the container body (1).

## Patentansprüche

1. Ein Augentropfer enthaltend einen Behälterkörper (1), der einen Flüssigkeitsspeicherraum darin ausgebildet hat, einen Stöpsel (12) zum Führen einer Flüssigkeit nach außerhalb des Behälterkörpers (1) und ein Einträufelöffnungselement (2), das in den Stöpsel (12) eingesetzt ist und geformt ist, um eine festgelegte Menge an medizinischer Flüssigkeit, die im Inneren des genannten Einträufelöffnungselementes (2) enthalten ist, durch eine Einträufelöffnung (21) abzugeben, die an einem Ende des Einträufelöffnungselementes (2) ausgebildet ist und die zylindrisch geformt ist und einen Deckel (3) hat, der darauf sitzt,
wobei der Augentropfer außerdem einen Siegelabschnitt (5) enthält zum hermetischen Abgrenzen von Flüssigkeit im Inneren des Behälterkörpers (1) und ein Entsiegelelement (6) zum Öffnen des Siegelabschnitts (5), und ein Klappenventil (4) das in dem Stöpsel (12) vorgesehen ist in einem Bereich, der näher zu dem Flüssigkeitsspeicherraum ist als das Sigelelement (5) und das Entsiegelelement (6), um dem Entsiegelelement (6) zu ermäglichen, den Siegelabschnitt (5) zu öffnen, wenn der Deckel (3) entlang einer axialen Richtung des Behälter niedergedrückt wird, um das Einträufelöffnungselement (2) in Richtung des Behälterkörpers (1) zu drücken und um das Einträufelöffrungselement (2) relativ zu dem Stöpsel (12) zu bewegen, während es verhindert, dass die Flüssigkeit zum Inneren des Behälterskörpers (1) zurückfließt.

2. Der Augentropfer gemäß Anspruch 1.
wobei das Entsiegelelement (6) den Siegelabschnitt (5) vom Behälterkorper (1) in Richtung des Einträufelöifnungselementes (2) durchsticht.

3. Der Augentropfer gemäß Anspruch 1,
wobei das Entsiegelelement (6) den Siegelabschnitt (5) vom Einträufelöffnungselement (2) in Richtung des Behälterkörpers (1) durchsticht.

4. Der Augentropfer gemäß einem der Ansprüche 1 bis 3, wobei der Behälterkörper (1) einen flexiblen Abschnitt (13) hat, um die Kapazität des Behälterkörpers (1) zu verändern, in Abhängigkeit von einer Menge an Flüssigkeit, die in dem Inneren des Behälterkörpers (1) enthalten ist

## Revendications

1. Compte-gouttes oculaire comprenant un corps de récipient (1) incluant un espace de stockage de liquide ménagé dans celui-ci, un bouchon (12) destiné à guider un liquide vers l'extérieur du corps de récipient (1) et un élément d'ouverture pour instillation (2) adapté dans le bouchon (12) et formé pour distiller une quantité fixée de liquide médical retenue à l'intérieur dudit élément d'ouverture pour instillation (2) à travers une ouverture pour instillation (21) ménagée au niveau d'une extrémité de l'élément d'ouverture pour instillation (2) et qui présente une forme cylindrique et comporte un capuchon (3) adapté sur celui-ci,
où le compte-gouttes oculaire comprend en outre une partie d'étanchéité (5) destinée à confiner de manière hermétique un liquide à l'intérieur du corps de récipient (1), un élément de non-étanchéité (6) destiné à ouvrir la partie d'étanchéité (5), et un clapet anti-retour (4) prévu dans le bouchon (12) et au niveau d'une partie plus proche de l'espace de stockage de liquide que de la partie d'étanchéité (5) et que de l'élément de non-étanchéité (6) en vue de permettre à l'élément de non-étanchéité (6) d'ouvrir la partie d'étanchéité (5) lorsque le capuchon (3) est comprime le long d'une direction axiale du récipient afin de pousser l'élément d'ouverture pour instillation (2) vers le corps de récipient (1), et de déplacer l'élément d'ouverture pour instillation (2) par rapport au bouchon (12), tout en empêchant le liquide de revenir à l'intérieur du corps de récipient (1).

2. Compte-gouttes selon la revendication 1, dans lequel l'élément de non-étanchéité (6) perce la partie d'étanchéité (5) depuis le corps de récipient (1) vers l'élément d'ouverture pour instillation (2).

3. Compte-gouttes selon la revendication 1, dans lequel l'élément de non-étanchéité (6) perce la partie d'étanchéité (5) depuis l'élément d'ouverture pour instillation (2) vers le corps de récipient (1).

4. Compte-gouttes selon l'une quelconque des revendications 1 à 3, dans lequel le corps de récipient (1) comporte une partie flexible (13) destinée à faire varier la capacité du corps de récipient (1) en réponse à une quantité de liquide contenue à l'intérieur du corps de récipient (1).
